(19) Europäisches Patentamt
European Patent Office
Office européen des brevets

(11) Publication number: **0 473 008 A2**

# (12) EUROPEAN PATENT APPLICATION

(21) Application number: **91113629.9**

(22) Date of filing: **14.08.91**

(51) Int. Cl.[5]: **C12P 35/04**, C12P 37/04, C12P 17/18

(30) Priority: **28.08.90 IT 2131590**

(43) Date of publication of application: **04.03.92 Bulletin 92/10**

(84) Designated Contracting States: **AT BE CH DE DK ES FR GB GR IT LI LU NL SE**

(71) Applicant: **RECORDATI S.A. CHEMICAL and PHARMACEUTICAL COMPANY**
**Corso S. Gottardo 54**
**CH-6830 Chiasso(CH)**

(72) Inventor: **Baldaro, Eva**
**Salita San Rocco, 15/B**
**I-16134 Genova(IT)**

(74) Representative: **Gervasi, Gemma, Dr. et al**
**Studio Brevetti e Marchi NOTARBARTOLO & GERVASI 33, Viale Bianca Maria**
**I-20122 Milano(IT)**

## (54) Improved process for preparing penicillins and cephalosporins.

(57) Improved process for preparing penicillins and cephalosporins by reacting 6-amino-penicillanic acid, 7-amino-cephalosporanicacid, 7-amino-3-deacetoxy-cephalosporanic acid or their derivatives with derivatives of α-aminoacids in the presence of a properly immobilised penicillin acylase enzyme, at a temperature ranging from -5 °C to +20 °C. The process minimises the production of by-products and ensures industrially advantageous yields.

EP 0 473 008 A2

The present invention relates to an industrially-advantageous improved process for the preparation of penicillins and cephalosporins of formula (I) and (II)

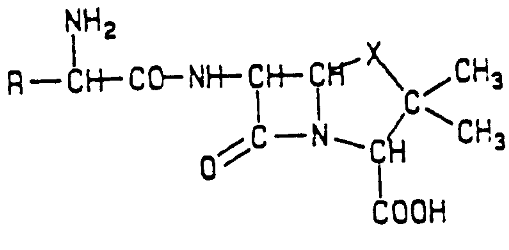

(I)

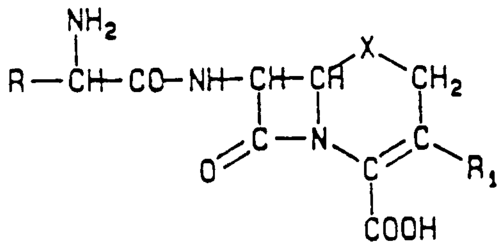

(II)

wherein X is an oxygen atom, a sulphur atom or a $CH_2$ group; R is a five or six-member hydrocarbon ring optionally substituted; $R_1$ is hydrogen atom, halogen atom, methyl group or a methylene group bonded to an organic radical via an oxygen, sulphur or nitrogen atom. This process consists of reacting an $\alpha$-substituted $\alpha$-aminoacid of formula (III)

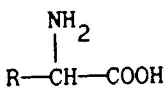

(III)

or a reactive derivative thereof, with a compound of formula (IV) or (V)

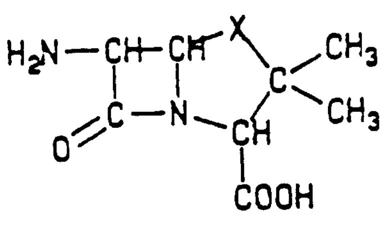

(IV)

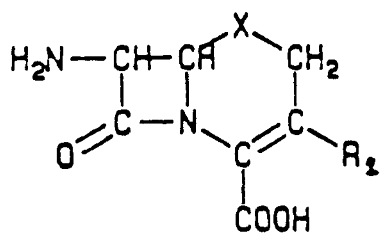

(V)

wherein X, R, and $R^1$ are the groups previously described, in the presence of an immobilised penicillin acylase enzyme at a temperature ranging from -5°C to +20°C.

The above reaction between reactive derivatives of $\alpha$-substituted $\alpha$-aminoacids and compounds derived from 7-aminocephalosporanic acid and 7-amino-deacetoxycephalosporanic acid is described in the U.S. patent No. 3,816,253, which specifies the course of the reaction in the presence of micro-organisms or enzymatic activities in aqueous medium at a temperature ranging from 5°C to 50°C. In particular, industrially advantageous yields are obtained at a temperature range of 20°C to 40°C.

Operating at the temperatures indicated in the above patent, the yield of the desired product can be reduced by concurrent parallel reactions which cause the formation of by-products that cannot always be easily separated from the reaction mixture and the presence of which will in any case affect the overall economics of the process.

The applicant has now found and it is an object of the present invention that it is possible to reduce drastically the formation of by-products and increase the yield of the desired product to industrially profitable levels by carrying out the above condensation reaction at lower temperatures ranging from +20°C to -5°C in the presence of immobilised penicillin acylase in a reaction medium made up of a suitable water/organic-solvent mixture, wherein the organic solvent ranges from 0 to 20% by volume.

The results obtained are totally surprising also in term of enzyme consumption: it is known, in fact, that the lowering of temperatures generally causes a slackening of enzymatic activity and, for this reason, in the process according to the present invention it is therefore necessary to initially increase the quantity of the enzyme used. However this does not cause an increase in the enzyme consumption, since the lower temperature will allow an extension of the enzyme service life. Thus, operating at temperatures ranging from 0°C to +10°C, it is possible to recycle the immobilized enzyme for several times, thereby minimising consumption.

It is accordingly an object of the present invention to provide an improved process for the preparation of penicillins and cephalosporins of formula (I) and (II), which process consists of reacting α-aminoacids of formula (III) or their reactive derivatives with compounds of formula (IV) or (V) in the presence of an immobilised penicillin acylase enzyme at a temperature ranging from -5°C to +20°C in an aqueous-organic medium.

With respect to the above formulas (I) and (II), R can be: unsubstituted phenyl, cyclohexadienyl, cyclohexenyl or cyclohexyl; or it can be one of the mentioned radicals containing one or more substituents selected among: hydroxyl, halogen, alkyl, alkoxyl, carboxyl, nitro, amino, and the like.

$R^1$, in turn, can be a hydrogen atom, a halogen atom, a methyl or a methylene group bonded to an alkoxy, an alkoxycarbonyl or to a 5 or 6-membered heterocyclic group containing 1 to 4 heteroatoms selected from O, S and N, optionally bonded to the methylene group via an atom of O, S or N, and optionally bearing as substituents one or more groups selected among: hydroxy, halogen, alkyl, alkoxy, carbonyl, carboxy, cyano, amino and the like.

With respect to the reactive derivatives of α-aminoacids having formula (III), as examples can be mentioned the methyl esters of D-phenylglycine, D-p-hydroxyphenylglycine, and D-1,4-cyclohexadien-1-yl-glycine.

Finally, the following acids are of particular interest among the compounds of formula (IV) and (V): 6-amino-penicillanic acid, 7-amino-cephalosporanic acid, 7-amino-3-deacetoxy-cephalosporanic acid, 7-amino-3-chloro-cephalosporanic acid.

The penicillin acylase enzyme used in the process according to the present invention may derive from any of the known microbial sources. Among these we may name micro-organisms of the Xanthomonas, Pseudomonas, Aeromonas, Escherichia, Arthrobacter, Corynebacterium and Bacillus genera.

The use of penicillin acylase deriving from Escherichia Coli ATCC 9637 was found to be particularly beneficial.

As indicated, the process according to the present invention is carried out in the presence of an immobilised enzyme. To this end, well known immobilisation techniques such as: absorption, ionic or covalent bond to polymer matrices, cross-linking, trapping in gel or fibres, microencapsulation or membrane reactors may be used.

The use of an enzyme incorporated into cellulose triacetate fibre structures or covalently bonded to polyacrylamidic resin was found to be of particular interest.

In particular, compound of formula (I) and (II) can be prepared causing reactive derivatives of formula (III) compounds in concentrations of 1 to 20%, preferably 4 to 10%, to react with compounds of formula (IV) or (V) in concentrations of 0.5 to 10%, preferably 2 to 5%.

The reaction may be carried out at a temperature ranging from -5°C to +20°C, even if a temperature of 4°C is preferred.

Optimum pH is generally 7, but satisfactory yields can be obtained at a pH ranging from 5 to 8.

The formation of penicillins and cephalosporins of formula (I) and (II) is normally checked by HPLC at the following conditions:

Column: MERCK HIBAR SELECT B.

Eluent: $CH_3CN$ 14%; $KH_2PO_4$ 0,05M, pH 2,5 for $H_3PO_4$, 86%.

The identification and the quantitative analysis of the reaction products are carried out by comparison with an internal reference standard.

Maximum yields are generally reached after a 2-hour reaction.

The following examples will better explain the characteristics and applicability of the invention, but shall in no way be a limitation thereof.

Starting materials are available as follows:

6-amino-penicillanic acid and 7-amino-3-deacetoxycephalosporanic acid, are commercially available (SIGMA). 7-amino-3-chloro-cephalosporanic acid can be prepared as described by R.R. Chauvette, D.A. Pennington in J.Med. Chem., 18, 403 (1975). D-phenylglycine and D-p-hydroxyphenylglycine can be prepared from the commercially available D-aminoacids (SIGMA) following usual esterification procedures (e.g. HClin MeOH).

**EXAMPLE 1**

2 g of D-phenylglycine methyl ester and 1 g of 7-amino-3-deacetoxycephalosporanic acid are dissolved in 50 ml of distilled water at pH 7 and added to 250 mg of Immobilised Penicillin Acylase (60 IU) in a temperature-controlled reactor provided with a filtering-baffle bottom drain. The solution is incubated with stirring at 37° C, maintaining a constant pH by adding 1M NaOH. After 2 hours, an HPLC assay reveals the presence of 24.7 mg/ml of 7-(D-α-aminophenyl-acetamido)-3-deacetoxycephalosporanic acid (yield 72.6%). The reaction mixture is filtered away and the immobilised enzyme is put back to react in the same conditions. After 25 hours, the residual activity of the enzyme is 44% of the original activity (half-life 22 hours).

**EXAMPLE 2**

2 g of D-phenylglycine methyl ester, 1 g of 7-amino-3-deacetoxycephalosporanic acid and 500 mg of Immobilised Penicillin Acylase (120 IU) are caused to react in the conditions of Example 1 at a temperature of 22° C. After 2 hours, an HPLC assay reveals the presence of 26.9 mg/ml of 7-(D-α-aminophenylacetamido)-3-deacetoxycephalosporanic acid (yield 79%). The reaction mixture is filtered away and the immobilised enzyme is put back to react in the same conditions. After 60 hours, the residual activity of the enzyme is 74% of the original activity (half-life 118 hours).

**EXAMPLE 3**

3 g of D-phenylglycine methyl ester, 1.5 g of 7-amino-3-deacetoxycephalosporanic acid and 2,810 mg of Immobilised Penicillin Acylase (675 IU) are caused to react in the conditions of Example 1 at a temperature of 4° C. After 2 hours, an HPLC assay reveals the presence of 45.9 mg/ml of 7-(D-α-aminophenylacetamido)-3-deacetoxy-cephalosporanic acid (yield 90%).

**EXAMPLE 4**

2 g of D-phenylglycine methyl ester, 1 g of 7-amino-3-deacetoxycephalosporanic acid and 1,875 mg of Immobilised Penicillin Acylase (450 IU) are caused to react in the conditions of Example 1 at a temperature of 4° C. After 2 hours, an HPLC assay reveals the presence of 29.5 mg/ml of 7-(D-α-aminophenylacetamido)-3-deacetoxycephalosporanic acid (yield 87%). The reaction mixture is filtered away and the immobilised enzyme is put back to react in the same conditions. After 60 hours, the residual activity of the enzyme shows no significant changes as compared to the original activity (half-life higher than 800 hours). The reaction mixture from two combined reactions is injected into a chromatographic column packed with 100 ml of Amberlite XAD-2 (Rohm & Haas). The column is washed with 200 ml of water, 500 ml of an water:methanol (3:1) mixture and then eluted by water:methanol (1:1). The eluate is evaporated at 30° C under vacuum to obtain 2 g of 7-(D-α-aminophenylacetamido)-3-deacetoxycephalosporanic acid. NMR (δ in DMSO-d6): 1.93 (3H, s, $CH_3$); 3.3 (2H, q AB, S-$CH_2$-); 4.96 (1H, d, S-CH=); 5.0 (1H, s, H-C-$NH_2$); 5.58 (1H, d, CH-CH-NH); 7.2-7.4 (5H, m, H-Ar).
$[\alpha]^{25}$ = +148.5

**EXAMPLE 5**

2 g of D-phenylglycine methyl ester, 1 g of 7-amino-3-deacetoxycephalosporanic acid and 1,875 mg of Immobilised Penicillin Acylase (450 IU) are caused to react in the conditions of Example 1 at a temperature of 37° C. The maximum yield is obtained after 18 minutes and an HPLC assay reveals the presence of 24.5 mg/ml of 7-(D-α-aminophenyl- acetamido)-3-deacetoxycephalosporanic acid (yield 72%).

**EXAMPLE 6**

2 g of D-phenylglycine methyl ester and 1 g of 7-amino-3-deacetoxycephalosporanic acid are dissolved in 40 ml of distilled water and 10 ml of ethylene glycol at pH 7 and added to 8.33 g of Immobilised Penicillin Acylase (2,000 IU). The solution is incubated with stirring at -5° C, maintaining a constant pH by adding 1M NaOH. After 2 hours, an HPLC assay reveals the presence of 30.2 mg/ml of 7-(D-α-aminophenylacetamido)-3-deacetoxycephalosporanic acid (yield 89%).

**EXAMPLE 7**

3 g of D-phenylglycine methyl ester, 1 g of 7-amino-3-deacetoxycephalosporanic acid and 500 mg of Immobilised Penicillin Acylase (120 IU) are caused to react in the conditions of Example 1 at a temperature of 22 °C. After 2 hours, an HPLC assay reveals the presence of 29.9 mg/ml of 7-(D-α-aminophenylacetamido)-3-deacetoxycephalosporanic acid (yield 88%).

**EXAMPLE 8**

3 g of D-phenylglycine methyl ester, 1 g of 7-amino-3-deacetoxycephalosporanic acid and 250 mg of Immobilised Penicillin Acylase (60 IU) are caused to react in the conditions of Example 1 at a temperature of 37 °C. After 2 hours, an HPLC assay reveals the presence of 25.2 mg/ml of 7-(D-α-aminophenylacetamido)-3-deacetoxycephalosporanic acid (yield 74%).

**EXAMPLE 9**

3 g of D-phenylglycine methyl ester, 1 g of 7-amino-3-deacetoxycephalosporanic acid and 1,875 mg of Immobilised Penicillin Acylase (450 IU) are caused to react in the conditions of Example 1 at a temperature of 4 °C. After 2 hours, an HPLC assay reveals the presence of 31.7 mg/ml of 7-(D-α-aminophenylacetamido)-3-deacetoxycephalosporanic acid (yield 93.3%).

**EXAMPLE 10**

3 g of D-phenylglycine methyl ester, 1.5 g of 7-amino-3-deacetoxycephalosporanic acid and 750 mg of Immobilised Penicillin Acylase (180 IU) are caused to react in the conditions of Example 1 at a temperature of 22 °C. After 2 hours, an HPLC assay reveals the presence of 41.8 mg/ml of 7-(D-α-aminophenylacetamido)-3-deacetoxy-cephalosporanic acid (yield 82%).

**EXAMPLE 11**

3 g of D-phenylglycine methyl ester, 1.5 g of 7-amino-3-deacetoxycephalosporanic acid and 375 mg of Immobilised Penicillin Acylase (90 IU) are caused to react in the conditions of Example 1 at a temperature of 37 °C. After 2 hours, an HPLC assay reveals the presence of 34.2 mg/ml of 7-(D-α-aminophenylacetamido)-3-deacetoxycephalosporanic acid (yield 67%).

**EXAMPLE 12**

0.63 g of D-phenylglycine methyl ester and 0.36 g of 7-amino-3-chlorocephalosporanic acid are dissolved in 18 ml of distilled water at pH 7 and added to 90 mg of Immobilised Penicillin Acylase (22 IU) in a temperature-controlled reactor provided with a filtering-baffle bottom drain. The solution is incubated with stirring at 37 °C, maintaining a constant pH by adding 1M NaOH. After 2 hours, an HPLC assay reveals the presence of 23.6 mg/ml of 7-(D-α-aminophenylacetamido)-3-chloro-cephalosporanic acid (yield 72%).

**EXAMPLE 13**

0.63 g of D-phenylglycine methyl ester, 0.36 g of 7-amino-3-chloro cephalosporanic acid and 540 mg of Immobilised Penicillin Acylase (130 IU) are caused to react in the conditions of Example 12 at a temperature of 4 °C. After 2 hours, an HPLC assay reveals the presence of 28.0 mg/ml of 7-(D-α-aminophenylacetamido)-3-chloro-cephalosporanic acid (yield 85%).

**EXAMPLE 14**

2 g of D-phenylglycine methyl ester, 1 g of 7-amino-3-deacetoxycephalosporanic acid and 60 IU of Free Penicillin Acylase Enzyme are caused to react in the conditions of Example 1 at a temperature of 37 °C. After 2 hours, an HPLC assay reveals the presence of 24.5 mg/ml of 7-(D-α-aminophenylacetamido)-3-deacetoxycephalosporanic acid (yield 72%).

**EXAMPLE 15**

2 g of D-phenylglycine methyl ester, 1 g of 7-amino-3-deacetoxycephalosporanic acid and 120 IU of Free Penicillin Acylase Enzyme are caused to react in the conditions of Example 1 at a temperature of 22° C. After 2 hours, an HPLC assay reveals the presence of 27.1 mg/ml of 7-(D-α-aminophenylacetamido)-3-deacetoxycephalosporanic acid (yield 79.8%).

**EXAMPLE 16**

2 g of D-phenylglycine methyl ester, 1 g of 7-amino-3-deacetoxycephalosporanic acid and 350 IU of Free Penicillin Acylase Enzyme are caused to react in the conditions of Example 1 at a temperature of 4° C. After 2 hours, an HPLC assay reveals the presence of 29.4 mg/ml of 7-(D-α-aminophenylacetamido)-3-deacetoxycephalosporanic acid (yield 86.6%).

**EXAMPLE 17**

1.9 g of D-phenylglycine methyl ester, 1 g of 6-aminopenicillanic acid and 250 mg of Immobilised Penicillin Acylase (60 IU) are caused to react in the conditions of Example 1 at a temperature of 37° C. After 2 hours, an HPLC assay reveals the presence of 11.3 mg/ml of 6-(D-α-aminophenylacetamido)-penicillanic acid (yield 35%).

**EXAMPLE 18**

1.9 g of D-phenylglycine methyl ester, 1 g of 6-aminopenicillanic acid and 500 mg of Immobilised Penicillin Acylase (120 IU) are caused to react in the conditions of Example 1 at a temperature of 22° C. After 2 hours, an HPLC assay reveals the presence of 14.2 mg/ml of 6-(D-α-aminophenylacetamido)-penicillanic acid (yield 44%).

**EXAMPLE 19**

1.9 g of D-phenylglycine methyl ester, 1 g of 6-aminopenicillanic acid and 1,875 mg of Immobilised Penicillin Acylase (450 IU) are caused to react in the conditions of Example 1 at a temperature of 4° C. After 2 hours, an HPLC assay reveals the presence of 16.5 mg/ml of 6-(D-α-aminophenylacetamido)-penicillanic acid (yield 51%).

**EXAMPLE 20**

2.1 g of D-p-hydroxyphenylglycine methyl ester, 1 g of 6-aminopenicillanic acid and 250 mg of Immobilised Penicillin Acylase (60 IU) are caused to react in the conditions of Example 1 at a temperature of 37° C. After 2 hours, an HPLC assay reveals the presence of 14.1 mg/ml of 6-(D-p-hydroxy-α-aminophenylacetamido)-penicillanic acid (yield 42%).

**EXAMPLE 21**

2.1 g of D-p-hydroxyphenylglycine methyl ester, 1 g of 6-aminopenicillanic acid and 500 mg of Immobilised Penicillin Acylase (120 IU) are caused to react in the conditions of Example 1 at a temperature of 22° C. After 2 hours, an HPLC assay reveals the presence of 17.2 mg/ml of 6-(D-p-hydroxy-α-aminophenylacetamido)-penicillanic acid (yield 51%).

**EXAMPLE 22**

2.1 g of D-p-hydroxyphenylglycine methyl ester, 1 g of 6-aminopenicillanic acid and 1,875 mg of Immobilised Penicillin Acylase (450 IU) are caused to react in the conditions of Example 1 at a temperature of 4° C. After 2 hours, an HPLC assay reveals the presence of 19.3 mg/ml of 6-(D-p-hydroxy-α-aminophenylacetamido)penicillanic acid (yield 57%).

**EXAMPLE 23**

2.1 g of D-p-hydroxyphenylglycine methyl ester, 1 g of 7-amino-3-deacetoxycephalosporanic acid and 250 mg of Immobilised Penicillin Acylase (60 IU) are caused to react in the conditions of Example 1 at a

temperature of 37° C. After 2 hours, an HPLC assay reveals the presence of 16.0 mg/ml of 7-(D-p-hydroxy-α-aminophenylacetamido)-3-deacetoxy cephalosporanic acid (yield 45%).

**EXAMPLE 24**

2.1 g of D-p-hydroxyphenylglycine methyl ester, 1 g of 7-amino-3-deacetoxycephalosporanic acid and 500 mg of Immobilised Penicillin Acylase (120 IU) are caused to react in the conditions of Example 1 at a temperature of 22° C. After 2 hours, an HPLC assay reveals the presence of 18.8 mg/ml of 7-(D-p-hydroxy-α-aminophenylacetamido)-3-deacetoxycephalosporanic acid (yield 53%).

**EXAMPLE 25**

2.1 g of D-p-hydroxyphenylglycine methyl ester, 1 g of 7-amino-3-deacetoxycephalosporanic acid and 1,875 mg of Immobilised Penicillin Acylase (450 IU) are caused to react in the conditions of Example 1 at a temperature of 37° C. After 2 hours, an HPLC assay reveals the presence of 21.0 mg/ml of 7-(D-p-hydroxy-α-aminophenylacetamido)-3-deacetoxycephalosporanic acid (yield 59%).

**Claims**

**1.** An improved process for the preparation of penicillins or cephalosporins having the general formula (I) or (II),

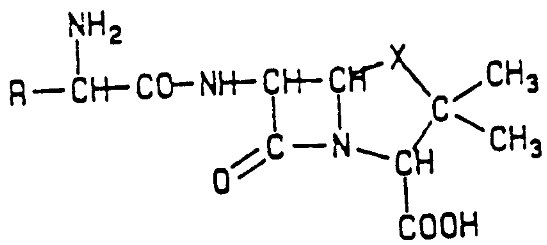

(I)

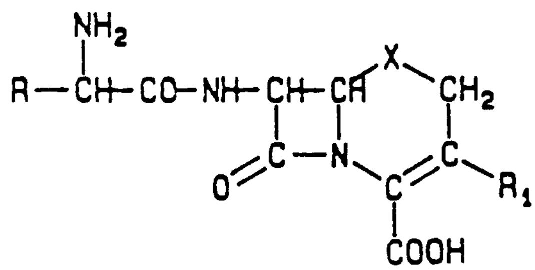

(II)

wherein: X is O, S or $CH_2$, R is a five or six-membered hydrocarbon ring optionally substituted and $R_1$ is a hydrogen atom, a halogen atom, a methyl group or a methylene group bonded to an organic radical via an atom of oxygen, sulphur or nitrogen; which process consists of reacting an α-substituted α-aminoacid of formula (III)

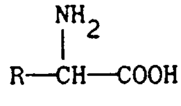

(III)

or a reactive derivative thereof, with a compound having the general formula (IV) or (V)

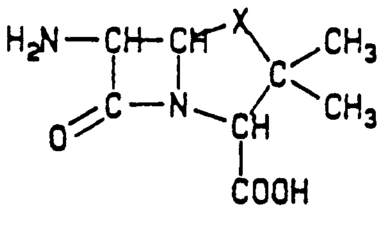

(IV)

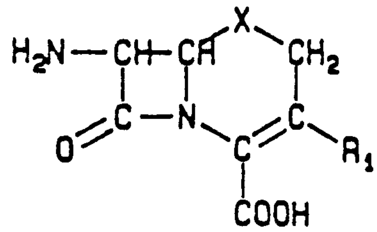

(V)

wherein: X, R and $R_1$ are defined as above, in the presence of an immobilised penicillin acylase enzyme at a temperature ranging from -5° C to 20° C.

**2.** The process according to claim 1, characterized in that penicillins or cephalosporins are produced in the presence of a immobilised penicillin acylase deriving from Escherichia Coli ATCC 9637.

**3.** The process according to claim 1, characterized in that penicillins or cephalosporins are produced in the presence of a penicillin acylase enzyme immobilised by a method selected among: absorption, ionic or covalent bond, polymer matrix, cross linking, trapping in gel or fibres, microencapsulation or membrane reactors.

**4.** The process according to claim 1, characterized in that penicillins or cephalosporins are produced in the presence of a penicillin acylase which has been immobilised preferably by trapping in cellulose triacetate fibre structures or bonded covalently to polyacrylamidic resins.

**5.** The process according to claim 1, characterized in that penicillins or cephalosporins are produced at a pH value ranging from 5 to 8.

**6.** The process according to claim 1, characterized in that penicillins or cephalosporins are produced in an appropriate water/organic solvent mixture, wherein the organic solvent is ranging from 0 to 20% in volume.

**7.** The process according to claim 1, characterized in that penicillins or cephalosporins are produced using an $\alpha$-substituted $\alpha$-aminoacid derivative in concentrations ranging from 1 to 20%.

**8.** The process according to the claim 1, characterized in that cephalosporins are produced using a derivative of an acid of formula (V) in concentration ranging from 0.5 to 10%.

**9.** The process according to claim 1, characterized in that penicillins or cephalosporins are produced using an $\alpha$-substituted $\alpha$-aminoacid derivative of formula (III) wherein R represents: phenyl, cyclohexenyl, cyclohexadienyl or cyclohexyl, each of them unsubstituted or containing one or more substituents selected among the group consisting of hydroxy, halogen, alkyl, alkoxy, carboxy, nitro or amino.

**10.** The process according to claim 1, characterized in that cephalosporins are produced using a compound of formula (V), wherein $R_1$ represents a hydrogen atom, a halogen atom, a methyl group or a methylene group bonded, via an atom of O, S or N, to an alkoxy, an alkoxycarbonyl or to a 5 or 6-membered heterocyclic group containing 1 to 4 heteroatoms selected from O, S and N, and optionally bearing as substituents one or more groups selected among: hydroxy, halogen, alkyl, alkoxy, carbonyl, carboxy, cyano and amino.

**11.** The process according to claim 1, characterized in that penicillins are produced using a derivative of the 6-amino penicillanic acid of formula (IV) in concentration ranging from 0.5 to 10%.